# EUROPEAN PATENT SPECIFICATION

(11) **EP 3 429 549 B1**
(45) Date of publication and mention of the grant of the patent: **20.04.2022**
(21) Application number: 17706172.8
(22) Date of filing: 10.02.2017
(51) Int. Cl.: A61J 15/00, A61B 5/00, A61B 5/06

(54) **FIBER-OPTIC REALSHAPE SENSING FEEDING TUBE**
ERNÄHRUNGSSONDE MIT FASEROPTISCHER REALSHAPE-ERFASSUNG
TUBE D'ALIMENTATION POUR DÉTECTION À FIBRE OPTIQUE DE FORME RÉELLE

(30) Priority: 15.03.2016 US 201662308243 P
(43) Date of publication of application: 23.01.2019
(73) Proprietor: Koninklijke Philips N.V., 5656 AG Eindhoven (NL)
(72) Inventor: FLEXMAN, Molly, Lar, 5656 AE Eindhoven (NL); KAHYA, Neriman, Nicoletta, 5656 AE Eindhoven (NL); VAN DER MARK, Martinus, Bernardus, 5656 AE Eindhoven (NL)
(74) Representative: Philips Intellectual Property & Standards
(86) International application number: PCT/EP2017/053023
(87) International publication number: WO 2017/157592

(56) References cited:
- EP-B1- 2 416 833
- WO-A1-2011/141830
- US-A1- 2014 275 997

## Description

### FIELD OF THE INVENTION

The present disclosure generally relates to a positioning of a feeding tube within an anatomical tract (e.g., a gastrointestinal tract or a vascular tract). The present disclosure specifically relates to a novel and inventive improvement of tracking the positioning of the feeding tube within the anatomical tract.

### BACKGROUND OF THE INVENTION

Generally, there two (2) feeding methods to addressing malnutrition patients who cannot maintain enough caloric intake orally. The first feeding method, known as an enteral nutrition, involves a positioning of a feeding tube within a gastrointestinal tract to provide nutritional and medical support to the patient. The second feeding method, known as a parenteral nutrition, involves a positioning of a feeding tube within a vein of the patient when the patient has a medical issue that makes safe use of the gastrointestinal tract difficult, or where adequate nutrition is not attained by enteral nutrition.

More specific to enteral nutrition, an access point for a feeding tube can be oral or nasal whereby a distal tip of the feeding tube is positioned in the stomach of the patient (i.e., a nasogastric enteral feeding), or positioned in an upper duodenum of the small intestine (i.e., a nasoduodenal enteral feeding), or positioned in a mid-jejunum of the small intestine (i.e., a nasojejunal enteral feeding). Optionally, a gastrostomy approach may be implemented to position the distal tip of the feeding tube in the stomach, or a jejunostomy approach may be implemented to position the distal tip of the feeding tube in the mid-jejunum of the small intestine.

Generally, there are three (3) placement methods for positioning the feeding tube within a gastrointestinal tract of a malnutrition patient. The first placement method, known as a blind placement method, involves positioning of the feeding tube the gastrointestinal tract without any navigational guidance. A problem with this placement method when orally or nasally accessing the patient is the feeding tube can often be mis-positioned with an airway of the patient, which can lead to pneumonia or pneumothorax.

A second placement method, known as an imaging tracking method, involves endoscopic or fluoroscopic imaging of the gastrointestinal tract for purposes of tracking the position of the feeding tube within the gastrointestinal tract. While safer and more successful than a blind placement approach, a problem with imaging tracking methods is a requirement of a medical specialist to perform the procedure. Consequently, the patient has to be transported from a clinical ward to an endoscopy department or a radiology department, which can lead to delays in feeding tube placement, additional complexity, and higher cost.

A third placement method, known as electromagnetic ("EM") tracking method, uses a stylet with an EM sensor at the tip that is placed within the feeding tube to thereby conduct a real-time tracking of a path of the feeding tube within the gastrointestinal tract. After the feeding tube has been navigated into position, the EM-guided stylet is withdrawn. The advantage of the EM tracking method is that it can be done at the patient bedside, thus providing a more patient-friendly and cost-effective solution as compared with fluoroscopy and endoscopic guidance. However, because EM tracking is limited to the tip of the feeding tube, EM tracking cannot fully represent the shape that the tube is taking on at any given time. This shape of the tube is important for detecting if **buckling** of the tube is occurring and if the tube has been placed correctly, especially in the presence of altered or shifting anatomy.

EP 2 416 833 B1 discloses an optically guided feeding tube with an optical system to enable directed placement and confirmation of correct placement in the gastrointestinal tract.

### SUMMARY OF THE INVENTION

The present disclosure improves upon prior feeding tube placement methods by providing inventions utilizing an integration of a Fiber-Optic RealShape ("FORS") sensor and a feeding tube to configure a FORS feeding tube for facilitating a tracking of a shape of the FORS feeding tube positioned within an anatomical tract (e.g., a gastrointestinal tract or a vascular tract).

For purposes of the inventions of the present disclosure, the term "Fiber-Optic RealShape ("FORS") sensor broadly encompasses an optical fiber structurally configured as known in the art for extracting high density strain measurements of the optical fiber derived from light emitted into and propagated through the optical fiber and reflected back within the optical fiber in an opposite direction of the propagated light and/or transmitted from the optical fiber in a direction of the propagated light.

An example of a FORS sensor includes, but is not limited to, one (1) or more optical fibers structurally configured under the principle of Optical Frequency Domain Reflectometry (OFDR) for extracting high density strain measurements of the optical fiber derived from light emitted into and propagated through the optical fiber(s) and reflected back within the optical fiber(s) in an opposite direction of the propagated light and/or transmitted from the optical fiber(s) in a direction of the propagated light via controlled grating patterns within the optical fiber (e.g., Fiber Bragg Gratings), a characteristic backscatter of the optical fiber(s) (e.g., Rayleigh backscatter) or any other arrangement of reflective element(s) and/or transmissive element(s) embedded, etched, imprinted, or otherwise formed in the optical fiber(s).

Commercially and academically, Fiber-Optic RealShape is also known as optical shape sensing ("OSS").

For purposes of the inventions of the present disclosure, the terms "feeding tube" is to be interpreted as understood in the art of the present disclosure and as exemplary described herein. Examples of feeding tubes include, but are not limited to, nasogastric feeding tubes, gastrostomy feeding tubes, jejunostomy feeding tubes, and esophagostomy feeding tubes.

The present disclosure relates to a FORS feeding tube system employing a feeding tube for channeling a fluid flow (e.g., a nutritional liquid, water and/or a liquid medicine) from a proximal end to a distal end of the feeding tube, and further employing a FORS sensor for generating sensing data informative of a shape reconstruction of a segment or an entirety of the FORS sensor. The feeding tube and the FORS sensor are integrated to configure a FORS feeding tube extending between the proximal end of the feeding tube and a distal end of the FORS sensor, and the sensing data is further informative of a shape of a segment or an entirety of the FORS feeding tube derived from the integration of the FORS sensor and the feeding tube. Additionally, the FORS sensor may be integrated into a positioning guide (e.g., a guidewire, a stylet or a tube) for facilitating an integration of the FORS sensor within the feeding tube. The FORS feed tube system may further employ a navigation controller to control a tracking of a shape of a segment or an entirety of the FORS feeding tube as positioned within an anatomical tract (e.g., a gastrointestinal tract).

Also discosed is a FORS feeding tube method (not part of the claims) involving a positioning of a segment or an entirety of a FORS feeding tube within an anatomical tract (e.g., a gastrointestinal tract), wherein the FORS feeding tube includes a integration of a feeding tube and a FORS sensor. The FORS feeding method further involves the FORS sensor generating sensing data informative of a shape reconstruction of a segment or an entirety of the FORS sensor, and further informative of a shape of a segment or an entirety of the FORS feeding tube derived from the integration of the feeding tube and the FORS sensor. The FORS feeding method may further involve a navigation controller controlling a tracking of the shape of the segment or the entirety of the FORS feeding tube as positioned within the anatomical tract.

For purposes of the inventions of the present disclosure, the terms "sensing data", "shape reconstruction", and "tracking" are to be interpreted as understood in the art of the present disclosure and as exemplary described herein.

For purposes of the inventions of the present disclosure, the term "integrate" and any tense thereof broadly encompasses any type of permanent or temporary adjoining, coupling, connecting, affixing, clamping, mounting, etc. of the FORS sensor and the feeding tube involving direct physical contact between the FORS sensor and the feeding tube or an adjacent placement of the FORS sensor and the feeding tube as understood in the art of the present disclosure and as exemplary described herein.

The term "integrate" and any tense thereof further broadly encompasses an integration of the FORS sensor and the feeding tube whereby a known alignment of a segment or an entirety of the FORS sensor and the feeding tube are for purposes of a shape tracking of the FORS feeding tube (e.g., a parallel alignment of a segment or an entirety of the longitudinal axes of the FORS sensor and the feeding tube as exemplary described herein).

For purposes of the present disclosure, the term "positioning guide" broadly encompasses any medical device utilized for guiding purposes as understood in the art of the present disclosure and as exemplary described herein. Examples of positioning guides include, but are not limited to, a guidewire, a stylet and a tube.

For purposes of the present disclosure, the term "controller" broadly encompasses all structural configurations of an application specific main board or an application specific integrated circuit housed within or linked to a workstation for controlling an application of various inventive principles of the present disclosure as subsequently described herein. The structural configuration of the controller may include, but is not limited to, processor(s), computer-usable/computer readable storage medium(s), an operating system, application module(s), peripheral device controller(s), slot(s) and port(s).

For purposes of the present disclosure, the label "navigation" used herein for the term "controller" distinguishes for identification purposes the navigation controller from other controllers as described and claimed herein without specifying or implying any additional limitation to the term "controller".

Examples of a "workstation" include, but are not limited to, an assembly of one or more computing devices, a display/monitor, and one or more input devices (e.g., a keyboard, joysticks and mouse) in the form of a client computer, a desktop or a tablet.

For purposes of the present disclosure, the term "application module" broadly encompasses a component of the workstation consisting of an electronic circuit and/or an executable program (e.g., executable software stored on non-transitory computer readable medium(s) and/firmware) for executing a specific application.

The foregoing forms and other forms of the inventions of the present disclosure as well as various features and advantages of the inventions of the present disclosure will become further apparent from the following detailed description of various embodiments of the inventions of the present disclosure read in conjunction with the accompanying drawings. The detailed description and drawings are merely illustrative of the inventions of the present disclosure rather than limiting, the scope of the inventions of the present disclosure being defined by the appended claims.

### BRIEF DESCRIPTION OF THE DRAWINGS

FIG. 1 illustrates exemplary FORS feeding tube placements in accordance with the inventive principles of the present disclosure.
FIG. 2 illustrates a general embodiment of a FORS feeding tube in accordance with the inventive principles of the present disclosure.
FIGS. 3A and 3B illustrate exemplary embodiments of a integration of a FORS sensor and a feeding tube in accordance with the inventive principles of the present disclosure.
FIGS. 4A-5F illustrate exemplary embodiments of a FORS sensor feeding tube in accordance with the inventive principles of the present disclosure.
FIG. 6 illustrates an exemplary embodiment of a FORS feeding tube system in accordance with the inventive principles of the present disclosure.
FIG. 7 illustrates a flowchart representative of an exemplary embodiment of a FORS feeding tube method in accordance with the inventive principles of the present disclosure.
FIGS. 8A-8C illustrate exemplary displays of a FORS feeding tube in accordance with the inventive principles of the present disclosure.

### DETAILED DESCRIPTION OF THE PREFERRED EMBODIMENTS

As an improvement upon prior feeding tube placement methods, the inventions of the present disclosure propose a Fiber-Optic RealShape ("FORS") sensing solution for facilitating an accurate placement of a feeding tube positioned within an anatomical tract. The FORS sensing solution provides a integration of a FORS sensor and a feeding tube to configure a FORS feeding tube for facilitating a tracking of a shape of a segment or an entirety of the FORS feeding tube positioned within an anatomical tract (e.g., a gastrointestinal tract or a vascular tract).

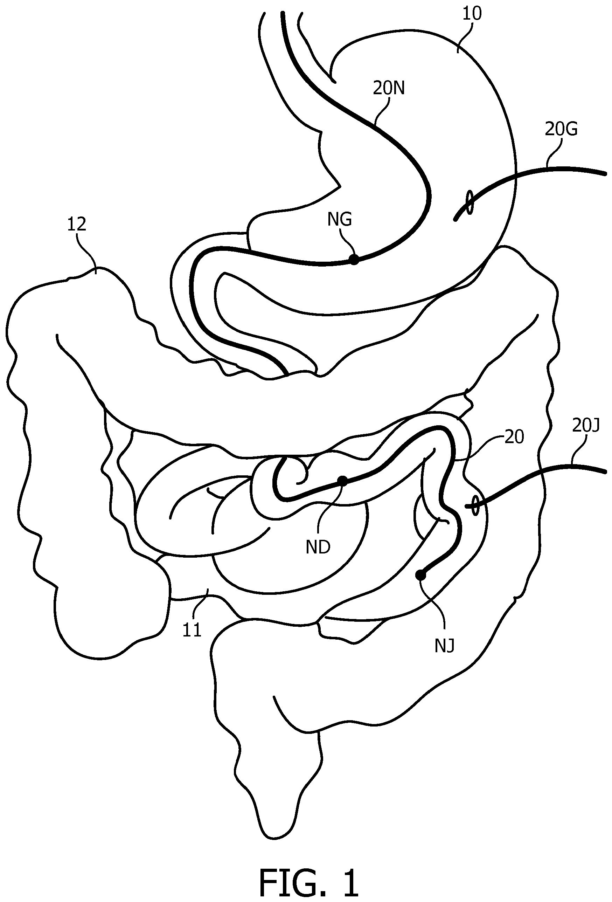

For example, referring to FIG. 1, various FORS sensors 20 of the present disclosure are shown positioned in a gastrointestinal tract including a stomach 10, a small intestine 11 and a large intestine 12. More particularly, a nasogastric FORS feeding tube 20N is shown for a nasogastric enteral feeding NG in stomach 10, a nasoduodenal enteral feeding ND in an upper duodenum of small intestine 11 or a nasojejunal enteral feeding NJ a mid-jejunum of small intestine 11. Additionally, a gastrostomy FORS feeding tube 20G is shown for a gastrostomy enteral feeding, and a jejunostomy FORS feeding tube 20J is shown for jejunostomy enteral feeding.

Still referring to FIG. 1, each FORS feeding tube 20 includes a FORS sensor providing three-dimensional ("3D") shape information of a feeding tube for improved placement of a FORS feeding tube 20 within the gastrointestinal tract. As will be further described herein, for navigation purposes, the shape of a FORS feeding tube 20 may be displayed (1) individually in space, (2) as an overlay on a live image (e.g., via a camera), and/or (3) as an overlay on a patient model. Additionally, as will be further described herein, the shape of a FORS feeding tube 20 may be analyzed to determine various metrics about important parameters including, but not limited to, an insertion length of a FORS feeding tube 20 within the gastrointestinal tract, a curvature-based prediction of a correct placement of a FORS feeding tube 20 within the gastrointestinal tract, and a measurement of physiologically-relevant parameters (e.g., breathing motion, internal body temperature, breathing, patient swallowing, patient movement, peristatic motion, coughing, etc.).

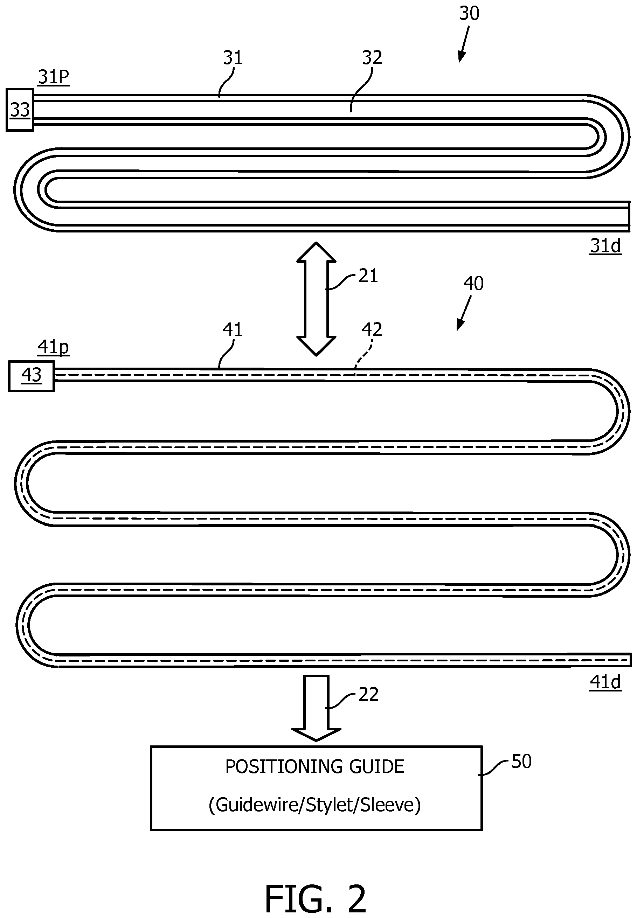

To facilitate an understanding of the various inventions of the present disclosure, the following description of FIGS. 2-5F teaches basic inventive principles of the present disclosure associated with an assembly and use of a FORS feeding tube of the present disclosure. From this description, those having ordinary skill in the art will appreciate how to apply the inventive principles of the present disclosure for making and using additional embodiments of a FORS feeding tube of the present disclosure. Please note the components of the present disclosure as shown in FIGS. 2-5F are not drawn to scale, but drawn to conceptually visualize the inventive principles of the present disclosure.

Referring to FIG. 2, a FORS feeding tube of the present disclosure employs a feeding tube 30 and a FORS sensor 40, and optionally employs a positioning guide 50.

Feeding tube 30 generally includes a tubular body 31 having a feeding lumen 32 for channeling a fluid flow (e.g., nutritional liquid, water and/or liquid medicine) from a proximal end 31p to a distal end 31d. Feeding tube 30 may further include a feeding luer 33, male or female, for connection to a source of the fluid flow (not shown). More particularly, in practice, feeding tube 30 is has dimensions suitable for a feeding a malnutrition patient. Examples of feeding tubes 30 include, but are not limited to, nasogastric feeding tubes, gastrostomy feeding tubes, jejunostomy feeding tubes, and esophagostomy feeding tubes.

FORS sensor 40 includes a single core or multi-core optical fiber 41 having controlled grating patterns (e.g., Fiber Bragg Gratings), a characteristic backscatter (e.g., Rayleigh backscatter) or any other arrangement of reflective elements and/or transmissive elements embedded, etched, imprinted, or otherwise formed in optical fiber 41. In practice, the controlled gratings, characteristic backscatter, or reflective/transmissive elements may extend along any segment or an entirety of optical fiber 41 as symbolically shown by dashed line 42 extending from a proximal end 41p to a distal end 41d. Also in practice, FORS sensor 40 may include of two (2) or more individual optical fibers 41 that may or may not be helixed.

In practice, optical fiber 41 of FORS sensor 40 may be made partially or entirely of any glass, silica, phosphate glass or other glasses, or made of glass and plastic or plastic, or other materials used for making optical fibers. For impeding any damage to FORS sensor 40 when introduced into the patient anatomy via manual or robotic insertion, each optical fiber 41 of FORS sensor 40 may be integrated into a positioning guide 50 via an embedding into a guide wire or a stylet as known in the art or permanently encircled by a protective sleeve as known in the art.

In practice, the protective sleeve may be made from any flexible material of a specified hardness including, but not limited to, pebax, nitinol, furcation tubing, and stranded metal tubing. Also in practice, the protective sleeve may consist of two or more tubular components of same or different degrees of flexibility and hardness in an overlapping and/or sequential arrangement.

FORS sensor 40 further includes an optical connector 43 for connecting optical fiber 41 to a launch or an optical source (e.g., optical integrator) as will be further described herein.

The inventions of the present disclosure are premised on an integration of feeding tube 30 and FORS sensor 40 to configure a FORS feeding tube for facilitating a tracking of a shape of a segment or an entirety of the FORS feeding tube as positioned within an anatomical tract. In practice, an integration of feeding tube 30 and FORS sensor 40 may be in any suitable configuration for a particular enteral feeding or a particular parental feeding.

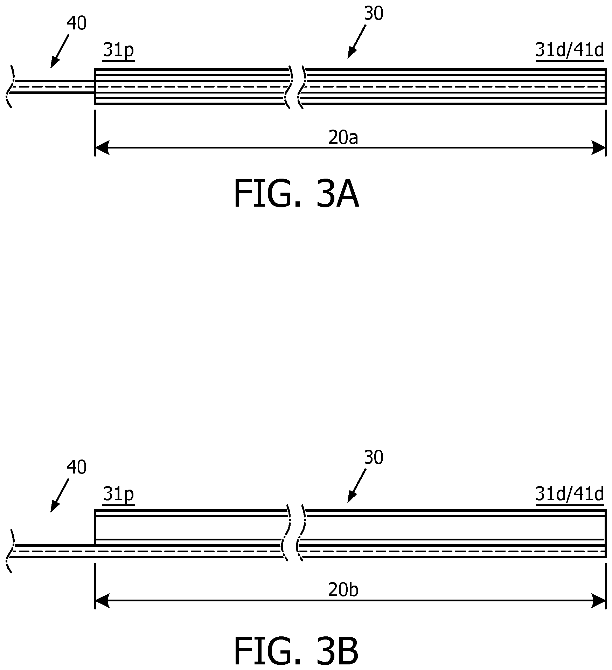

For example, as shown in FIG. 3A, a first integration approach of the present disclosure is to insert FORS sensor 40 into feeding lumen 32 of feeding tube 30 whereby proximal end 31p of feeding tube 30 and distal end 41d of FORS sensor define a FORS feeding tube 20a inclusive of proximal end 31p and/or distal end 41d, or exclusive of proximal end 31p and distal end 41d. In practice, the insertion may be permanent or temporary, and may partially or fully extend within feeding lumen 32 as shown.

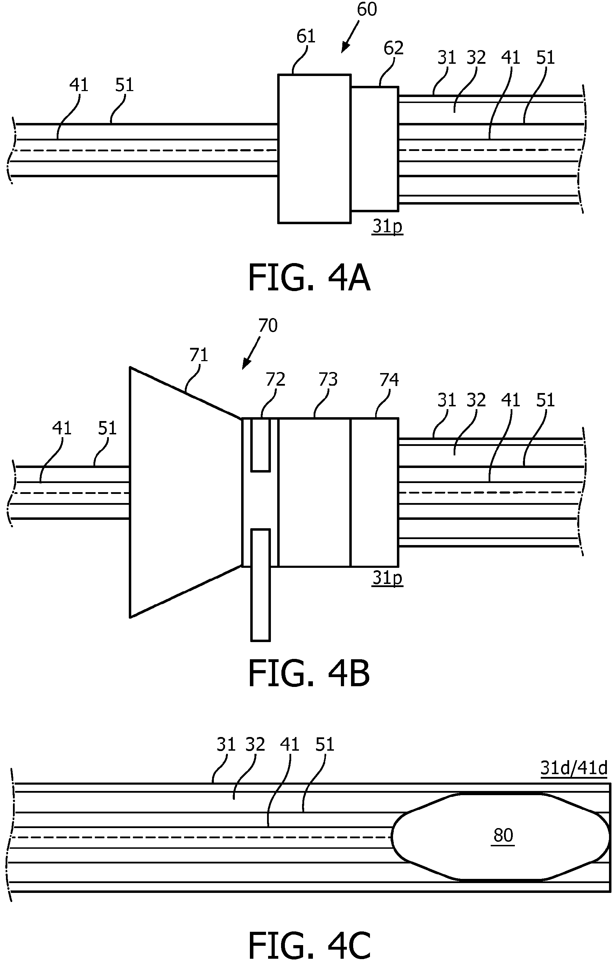

FIG. 4A illustrates a first exemplary embodiment of FIG. 3A with optical fiber 41 of FORS sensor 40 embedded into a guidewire 51, and being partially or fully inserted into feeding lumen 32 of feeding tube 30. A proximal FORS sensor lock 60 for temporarily fixing the insertion of optical fiber 41 and guidewire 51 into feeding lumen 32 includes a hub 61 attached to guidewire 51 and a locking luer 62 (which may also serve as feeding luer 33 of FIG. 2).

Alternatively, a duel luer lock may be utilized to allow for insertion of optical fiber 41 and guidewire 51 as well an injection of saline or other fluids.

FIG. 4B illustrates a second exemplary embodiment of FIG. 3A with optical fiber 41 of FORS sensor 40 embedded into guidewire 51, and being partially or fully inserted into feeding lumen 32 of feeding tube 30. A proximal FORS sensor lock 70 attached to feeding tube 30 includes a guiding funnel 71, a guidewire clamp 72 (e.g., a shaft collar, a set screw, a clip, etc.), a female luer 73 and a male luer 74 (which may also serve as feeding luer of FIG. 2). Lock 70 temporarily fixes the insertion of optical fiber 41 and guidewire 51 into feeding lumen 32.

FIG. 4C illustrates a third exemplary embodiment of FIG. 3A with optical fiber 41 of FORS sensor 40 embedded into guidewire 51, and fully inserted into feeding lumen 32 of feeding tube 30. Guidewire 51 includes a balloon 80 shown inflated to temporarily or permanently fix the insertion of feeding optical fiber 41 and guidewire 51 into feeding lumen 32 of feeding tube 30.

By further example, as shown in FIG. 3B, a second integration approach of the present disclosure is to attach or mount optical fiber 41 of FORS sensor 40 along an exterior of feeding tube 30 whereby proximal end 31p of feeding tube 30 and distal end 41d of FORS sensor 40 define a FORS feeding tube 20b inclusive of proximal end 31p and/or distal end 41d, or exclusive of proximal end 31p and distal end 41d. In practice, the attachment or mounting of optical fiber 41 may be permanent or temporary, and may partially or fully extend along feeding tube 30.

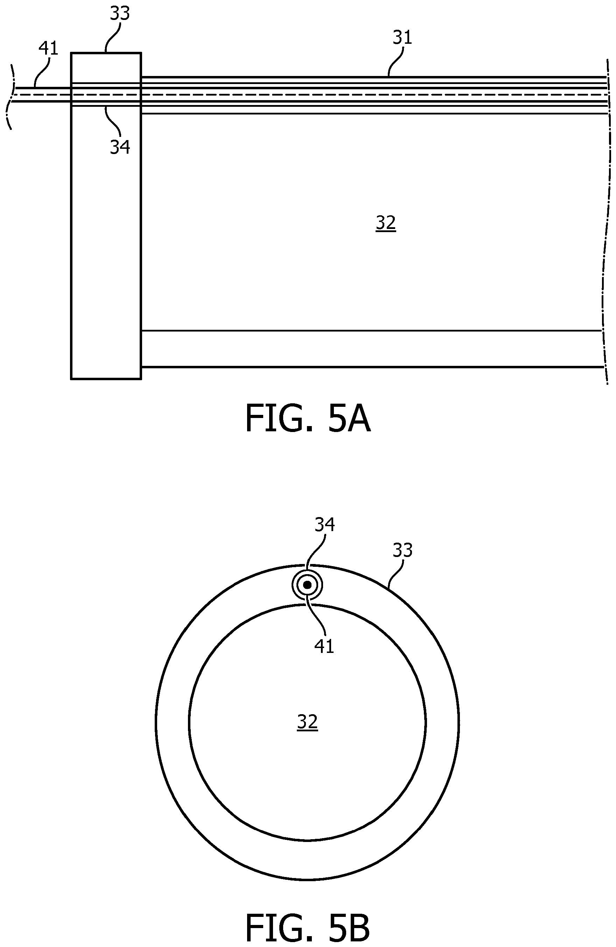

FIGS. 5A and 5B illustrate a first exemplary embodiment of FIG. 3B with optical fiber 41 of FORS sensor 40 being partially or fully inserted into a navigation lumen 34 of feeding tube 30 extending through a wall of tubular body 31 and feeding luer 33.

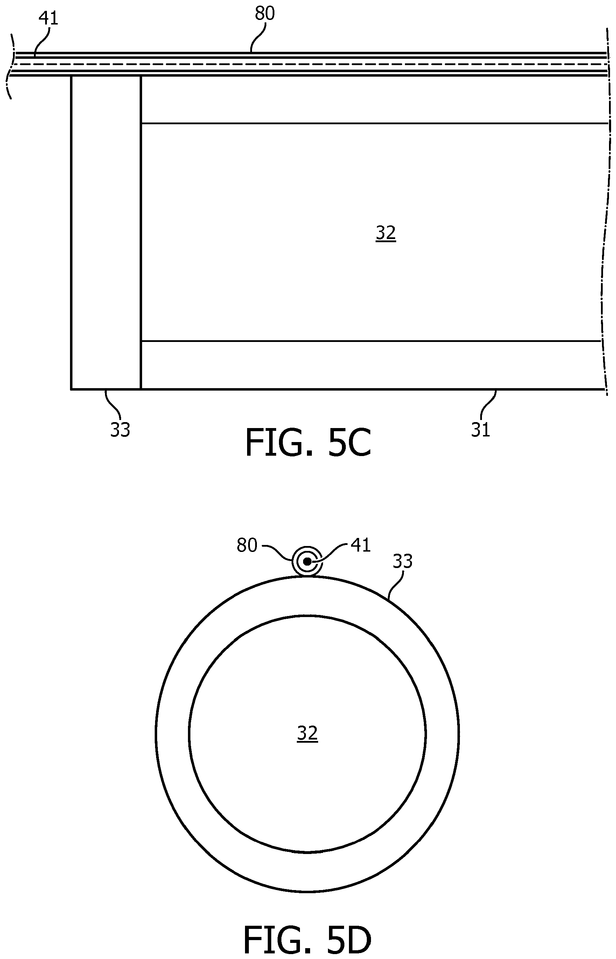

FIGS. 5C and 5D illustrate a second exemplary embodiment of FIG. 3B with optical fiber 41 of FORS sensor 40 being partially or fully inserted into a protective covering 80 attached or mounted to an exterior of tubular body 31 and feeding luer 33.

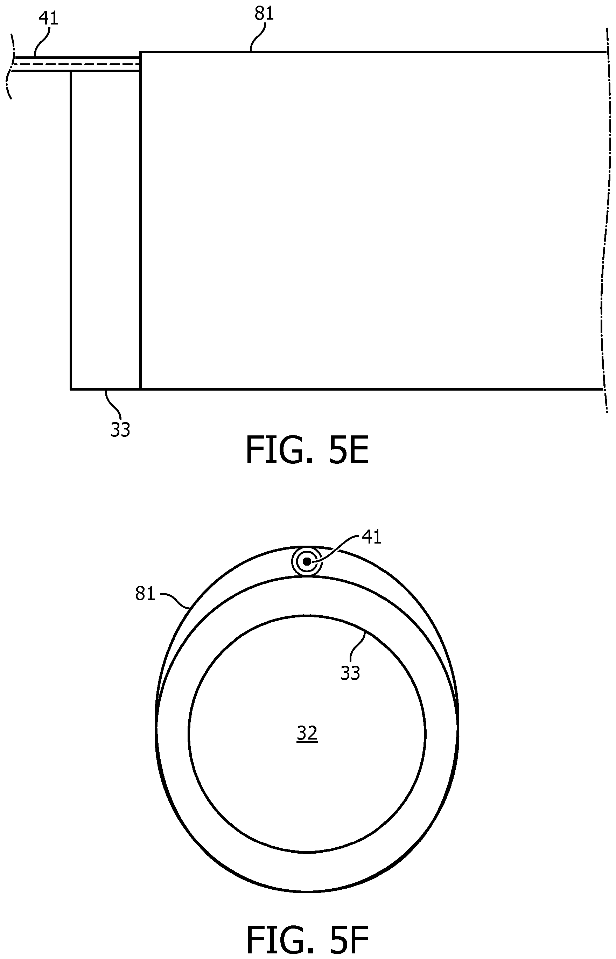

FIGS. 5E and 5F illustrates a third exemplary embodiment of FIG. 3B with optical fiber 41 of FORS sensor 40 attached to an exterior of tubular body 31 of feeding tube 30, and a protective covering 81 encircling optical fiber 41 and tubular body 31.

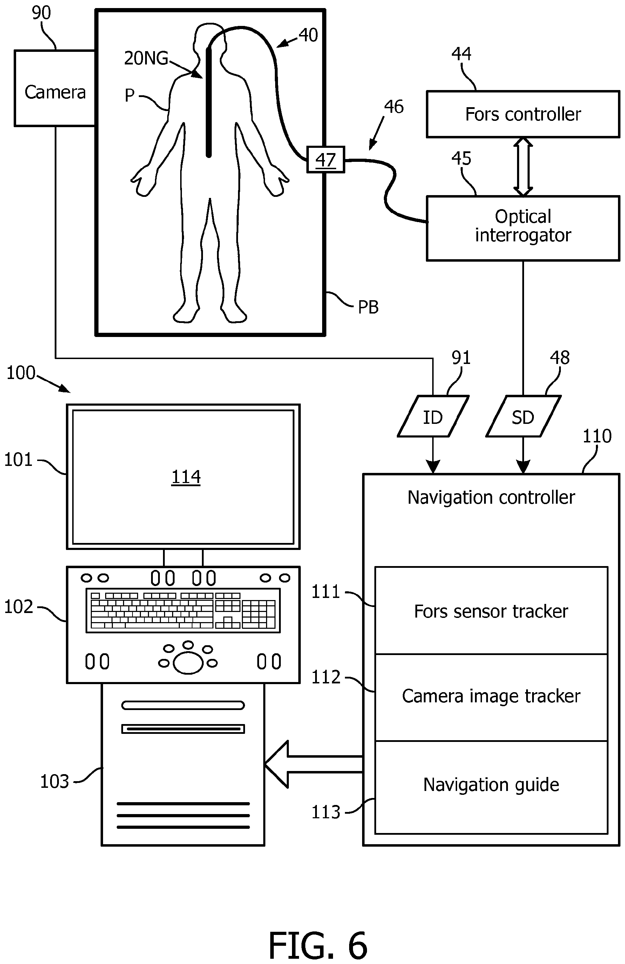

To facilitate a further understanding of the various inventions of the present disclosure, the following description of FIGS. 6-8 teaches basic inventive principles associated with FORS feeding tube systems and methods of the present disclosure. From this description, those having ordinary skill in the art will appreciate how to apply the inventive principles of the present disclosure for making and using additional embodiments of FORS feeding tube systems and methods of the present disclosure. Please note the components of the present disclosure as shown in FIGS. 6-8 are not drawn to scale, but drawn to conceptually visualize the inventive principles of the present disclosure.

Referring to FIG. 6, a FORS feeding tube system of the present disclosure employs a nasogastric FORS feeding tube 20NG, a workstation 100 and a navigation controller 110 for executing a FORS feeding tube method of the present disclosure involving a patient P lying prone on a patient bed PB.

In operation, a FORS sensor 40 of nasogastric FORS feeding tube 20NG distally extends from launch 47 adjoined to a rail of patient bed PB as shown or alternatively adjoined to a cart (not shown) next to patient bed PB or adjoined to a workstation (e.g., workstation 100 or a tablet (not shown)). An optical fiber 46 proximally extends from launch 47 to an optical integrator 45. In practice, optical fiber 46 may be a separate optical fiber connected to FORS sensor 40 at launch 47, or a proximal extension of FORS sensor 40 extending through launch 47.

As known in the art, a FORS controller 44 controls an emission of light by optical interrogator 45 via optical fiber 46 into FORS sensor 40 whereby the light is propagated through FORS sensor 40 to a distal end within the feeding tube to generate sensing data 48 informative of shape reconstruction of a segment or an entirety of FORS sensor 40 relative to launch 47 serving as a fixed reference position. In practice, the distal end of FORS sensor 40 may be closed, particularly for light reflective embodiments of FORS sensor 40, or may be opened, particularly for light transmissive embodiments of FORS sensor 40.

Navigation controller 110 is installed within workstation 100 including a known arrangement of a monitor 101, a keyboard 102 and a computer 103.

Navigation controller 110 includes application modules in the form of a FORS sensor tracker 111, an optional camera image tracker 112 and a navigation guide 113.

With FORS sensor 40 being connected to launch 47, FORS sensor tracker 111 processes sensing data 48 to track a shape of a segment or an entirety of nasogastric FORS feeding tube 20NG relative to launch 47 as known in the art.

Additionally, FORS sensor tracker 111 may analyze the shape of nasogastric FORS feeding tube 20NG to determine various metrics about important parameters including, but not limited to, an insertion length of a nasogastric FORS feeding tube 20NG within the gastrointestinal tract, and a measurement of physiologically-relevant parameters (e.g., breathing motion, internal body temperature, breathing, patient swallowing, patient movement, peristatic motion, coughing, etc.). Additionally, FORS sensor tracker 111 may further process shape parameters and/or metrics to predict the likelihood of correct placement of nasogastric FORS feeding tube 20NG within the gastrointestinal tract.

More particularly, FORS sensor tracker 111 may perform a measurement of a length of nasogastric FORS feeding tube 20NG inserted within the gastrointestinal tract by detecting the FORS axial strain change caused by the temperature change from room temperature to 37 °C after nasogastric FORS feeding tube 20NG enters patient P.

Furthermore, FORS sensor tracker 111 may perform a prediction of a position of nasogastric FORS feeding tube 20NG inserted within the gastrointestinal tract by using curvature along the shape and the length of insertion (e.g., a specific bend occurring ~ 10cm into feed tube 20NG representing a correct positioning of feed tube 20NG into the patient's esophagus as opposed to the patient's lungs).

In addition, FORS sensor tracker 111 may implement an automatic detection of a degree of respiratory motion to detect if nasogastric FORS feeding tube 20NG is misplaced in a lung of patient P. This could also be extended to include detecting sphincter locations.

The FORS feeding tube system optionally employs a camera 90 for generating image data 91 illustrative of still images and/or a live video of patient P as nasogastric FORS feeding tube 20NG is being manually or robotically positioned within a gastrointestinal tract of patient P as known in the art. If camera 90 is employed by the system, then camera image tracker 112 processes image data 90 for a guidance display 114 on monitor 101.

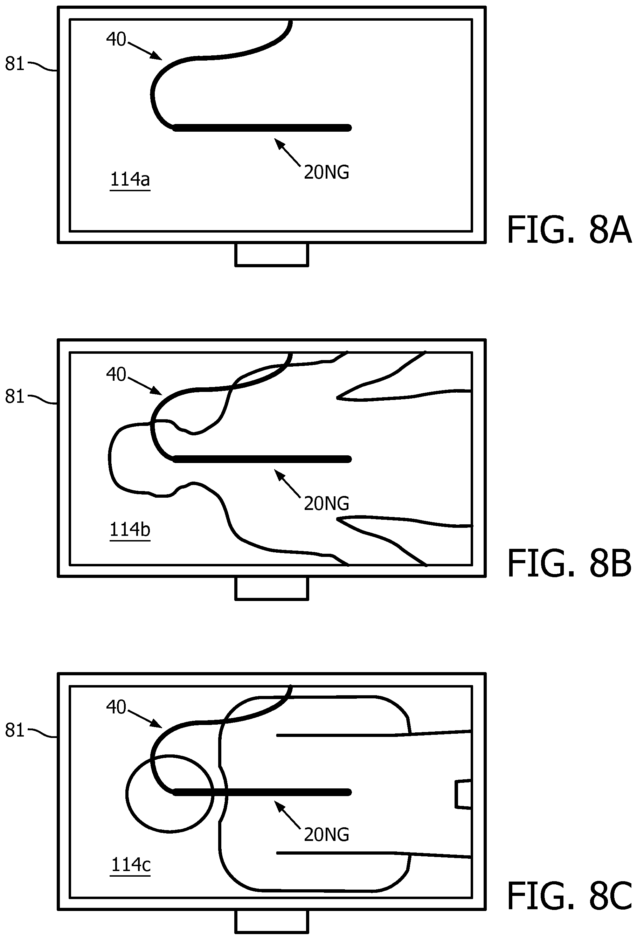

Based on the processing of sensing data 49 and imaging data 91, navigation guide 113 controls guidance display 114 of the shape of nasogastric FORS feeding tube 20NG in any suitable form for navigation purposes. A first exemplary form is a guidance display 114a of the shape of a segment or an entirety of nasogastric FORS feeding tube 20NG in space as exemplary shown in FIG. 8A. A second exemplary form is a guidance display 114b of the shape of a segment or an entirety of nasogastric FORS feeding tube 20NG as an overlay on a camera image or video of patient P as exemplary shown in FIG. 8B. A third exemplary form is a guidance display 114C of the shape of nasogastric FORS feeding tube 20NG as an overlay on an anatomical model as exemplary shown in FIG. 8C. The anatomical model may be enhanced to show locations of the lungs, stomach, small intestine, etc.

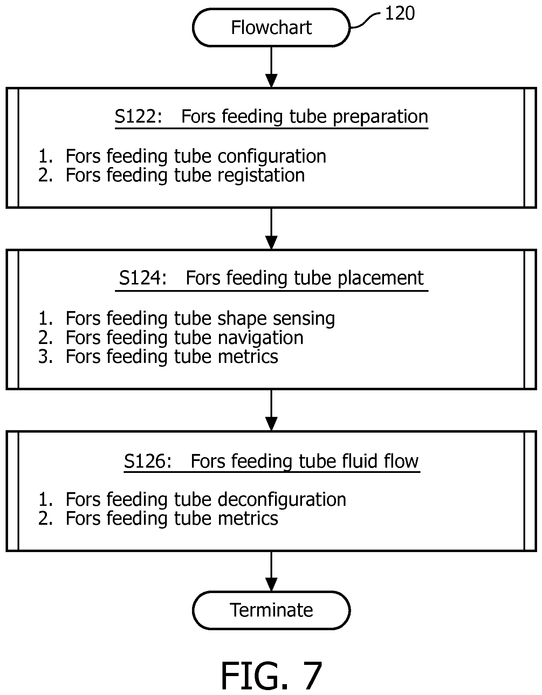

Referring to FIG. 7, a flowchart 120 representative of an exemplary FORS feeding tube method of the present disclosure will now be described herein in the context of nasogastric FORS feeding tube 20NG of FIG. 6 having proximal FORS sensor lock 70 of FIG. 4B for temporarily fixing FORS sensor 40 within feeding tube 30. From this description, those having ordinary skill in the art will appreciate how to apply the method to other embodiments of a FORS feeding tube of the present disclosure.

Referring to FIGS. 6 and 7, a stage S122 of flowchart 120 encompasses a preparation of nasogastric FORS feeding tube 20NG for manual or robotic placement within the gastrointestinal tract during a stage S124 of flowchart 120.

In one embodiment of stage S122, nasogastric FORS feeding tube 20NG is constructed after sterilization by a clamping via proximal FORS sensor lock 70 of a full insertion of FORS sensor 40 within feeding tube 30. Next, nasogastric FORS feeding tube 20NG is registered on dependence of the particular display 114 of the shape of a segment or an entirety of nasogastric FORS feeding tube 20NG within the gastrointestinal tract.

For guidance display 114a as shown in FIG. 8A, navigation guide 113 utilizes a template or directs an operator to position a tip of nasogastric FORS feeding tube 20NG at a head, a left shoulder, and a right shoulder to thereby register nasogastric FORS feeding tube 20NG in space.

For guidance display 114b as shown in FIG. 8B, navigation guide 113 directs a registration between the shape of a segment or an entirety of nasogastric FORS feeding tube 20NG and camera 90 by having launch 47 mounted on camera 90 or in a fixed relationship to camera 90. Alternatively, registration may be done by holding the shape of a segment or an entirety of nasogastric FORS feeding tube 20NG in the view of camera 90, and performing manual, semi-automatic, or automatic segmentation of the shape of nasogastric FORS feeding tube 20NG in the camera still image or video. Point based registration can also be used, as is known in the art.

For guidance display 114c as shown in FIG. 8C, navigation guide 113 directs a registration of the shape of a segment or an entirety of nasogastric FORS feeding tube 20NG to an anatomical model by pointing to standard anatomical landmark(s) as known in the art (e.g., a top of the head, a nose, a mouth, a solar plexus, a belly button, the nipples, etc.). In practice, different models may be selected or a standard model may be morphed to scale to the anatomical landmarks.

Still referring to FIGS. 6 and 7, in one embodiment of stage S124, FORS sensor tracker 111 initiates a generation of sensing data 48 as nasogastric FORS feeding tube 20NG is manually or robotically navigated into the gastrointestinal tract. If camera 90 is employed, a single camera feed may be utilized or a biplane view (top and side shown simultaneously), or a multi-camera view. The camera feed gives online anatomical references to the operator while introducing nasogastric FORS feeding tube 20NG into the gastrointestinal tract. Alternatively, static images may be acquired at the start of stage S124 using camera 90.

More particularly, in practice, virtual reality or augmented reality ("AR") may be implemented for a display of a position of nasogastric FORS feeding tube 20NG. For example, a shape of a segment or an entirety of nasogastric FORS feeding tube 20NG may be projected directly onto patient P, or may be shown to the user through AR glasses, or may be superimposed on a live image stream from a tablet or smartphone. For virtual reality, the user may see a model of patient P and then be able to move along nasogastric FORS feeding tube 20NG and see the pathway that it is taking.

Prior to, during and/or subsequent to nasogastric FORS feeding tube 20NG is manually or robotically navigated into the gastrointestinal tract, FORS sensor tracker 111 may determine various metrics as previously described herein. Of particular importance is a determination of insertion depth and tip location.

Still referring to FIGS. 6 and 7, upon targeted placement of nasogastric FORS feeding tube 20NG within the gastrointestinal tract, a stage S126 of flowchart 120 encompasses a fluid flow through nasogastric FORS feeding tube 20N to the gastrointestinal tract. In one embodiment of stage S126, FORS sensor 40 is unclamped and removed from feeding tube 30, which remains in the gastrointestinal tract to facilitate the delivery of the fluid. In an alternative embodiment of stage S126, for embodiments of FORS feeding tubes 20 of the present disclosure employing a permanent integration of a FORS sensor 40 and a feeding tube 30, FORS sensor tracker 111 may continue to determine various metrics during the delivery of the fluid, particularly a measurement of physiologically-relevant parameters (e.g., breathing motion, internal body temperature, breathing, patient swallowing, patient movement, peristatic motion, coughing, etc.).

Flowchart 120 is terminated upon manual or robotic removal of nasogastric FORS feeding tube 20NG from within the gastrointestinal tract.

Referring to FIGS. 1-8C, those having ordinary skill in the art will appreciate numerous benefits of the inventions of the present disclosure including, but not limited to, an accurate representation of a shape of a feeding tube within an anatomical tract at any given time to thereby detect any buckling of the feeding tube within the anatomical tract and determine any misplacement of the feeding tube within the anatomical tract, especially in the presence of altered or shifting anatomy.

Further, as one having ordinary skill in the art will appreciate in view of the teachings provided herein, features, elements, components, etc. described in the present disclosure/specification and/or depicted in the Figures may be implemented in various combinations of hardware and software, and provide functions which may be combined in a single element or multiple elements. For example, the functions of the various features, elements, components, etc. shown/illustrated/depicted in the Figures can be provided through the use of dedicated hardware as well as hardware capable of executing software in association with appropriate software for added functionality. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Having described preferred and exemplary embodiments of Fiber Optical RealShape feeding tubes, systems and methods, (which embodiments are intended to be illustrative and not limiting), it is noted that modifications and variations can be made by persons skilled in the art in light of the teachings provided herein, including the Figures. It is therefore to be understood that changes can be made in/to the preferred and exemplary embodiments of the present disclosure.

Moreover, it is contemplated that corresponding and/or related systems incorporating and/or implementing the device/system or such as may be used/implemented in/with a device in accordance with the present disclosure are also contemplated. Further, corresponding and/or related method for manufacturing and/or using a device and/or system in accordance with the present disclosure are also contemplated.

## Claims

1. An optical shape sensing (or "OSS") feeding tube system, comprising:
a feeding tube (30) including a feeding lumen for channeling a fluid flow from a proximal end of the feeding tube (30) to a distal end of the feeding tube (30);
an OSS sensor (40) for generating sensing data informative of a shape reconstruction of at least a segment of the OSS sensor (40);
wherein the feeding tube (30) and the OSS sensor (40) are integrated to configure a OSS feeding tube (20) extending between the proximal end of the feeding tube (30) and a distal end of the OSS sensor (40); and
wherein the sensing data is further informative of a shape of at least a segment of the OSS feeding tube (20) derived from integration of the OSS sensor (40) and the feeding tube (30).

2. The OSS feeding tube system of claim 1, further comprising:
a positioning guide (50) integrated with the OSS sensor (40);
wherein the OSS sensor (40) and the positioning guide (50) are at least partially insertable or partially inserted into the feeding lumen; and
wherein at least one of:
the feeding tube (30) further including a proximal OSS sensor lock (60, 70) operable to fix or fixing insertion of the OSS sensor (40) and the positioning guide (50) within the feeding lumen; and
the positioning guide (50) including a distal OSS sensor (40) lock operable to fix or fixing insertion of the OSS sensor (40) and the positioning guide (50) within the feeding lumen.

3. The OSS feeding tube system of claim 1, wherein the OSS sensor (40) includes an optical fiber (41); the system further comprising a guidewire (51) embedding said optical fiber (41), and fully inserted into feeding lumen (32) of feeding tube (30); the guidewire (51) comprising a balloon (80) inflatable or inflated to temporally or permanently fix the insertion of said optical fiber (41) and guidewire (51) into feeding lumen (32).

4. The OSS feeding tube system of claim 1,
wherein the feeding tube (30) further includes a navigation lumen; and
wherein the OSS sensor (40) is positionable or is positioned within the navigation lumen.

5. The OSS feeding tube system of claim 1, further comprising:
a protective sleeve integrated with an exterior of the feeding tube (30),
wherein the OSS sensor (40) is at least partially insertable into the protective sleeve.

6. The OSS feeding tube system of claim 1, further comprising:
a protective sleeve,
wherein the feeding tube (30) and the OSS sensor (40) are at least partially insertable into the protective sleeve.

7. The OSS feeding tube system of claim 1, further comprising:
a navigation controller operable, responsive to a generation of the sensing data by the OSS sensor (40), to control a tracking of the shape of the at least segment of the OSS feeding tube (20) as positioned within an anatomical tract,
wherein a tracking by the navigation controller of the shape of the OSS feeding tube (20) as positioned within the anatomical tract includes at least one of:
the navigation controller operable to control a display of the shape of the at least the segment of the OSS feeding tube (20) as positioned within the anatomical tract; and
the navigation controller operable to control a determination of at least one metric associated with the OSS feeding tube (20) as positioned within the anatomical tract.

8. The OSS feeding tube system of claim 7, wherein the control by the navigation controller of the display of the shape of the at least the segment of the OSS feeding tube (20) as positioned within the anatomical tract includes at least one of:
a display of the of the shape of the at least the segment of the OSS feeding tube (20) in space;
a display of the shape of the at least the segment of the OSS feeding tube (20) as an overlay on a patient image registered with the OSS feeding tube (20); and
a display of the shape of the at least the segment of the OSS feeding tube (20) as an overlay on a patient model registered with the OSS feeding tube (20).

9. The OSS feeding tube system of claim 7, wherein the control by the navigation controller of the determination of the at least one metric associated with the OSS feeding tube (20) as positioned within the anatomical tract includes at least one of:
a determination of an insertion length of the at least the segment the OSS feeding tube (20) as positioned within the anatomical tract;
a prediction of a correct placement of nasogastric the at least the segment the OSS feeding tube (20) as positioned within the anatomical tract; and
a measurement of physiologically-relevant parameters associated with OSS sensor (40).

## Patentansprüche

1. Ein optisches Formerfassungssystem (oder "OSS") mit Ernährungssonde, das Folgendes umfasst:
eine Ernährungssonde (30) mit einem Ernährungslumen zum Leiten eines Flüssigkeitsstroms vom proximalen Ende der Ernährungssonde (30) zum distalen Ende der Ernährungssonde (30);
einen OSS-Sensor (40) zum Generieren von Erfassungsdaten, die die Formrekonstruktion mindestens eines Segments des OSS-Sensors (40) angeben;
wobei die Ernährungssonde (30) und der OSS-Sensor (40) so integriert sind, dass sie eine OSS-Ernährungssonde (20) bilden, die sich zwischen dem proximalen Ende der Ernährungssonde (30) und dem distalen Ende des OSS-Sensors (40) erstreckt; und
wobei die Erfassungsdaten zudem Informationen über die Form mindestens eines Segments der OSS-Ernährungssonde (20) enthalten, die aus dem Integrieren des OSS-Sensors (40) und der Ernährungssonde (30) abgeleitet werden.

2. Das OSS-Ernährungssondensystem gemäß Anspruch 1, das zudem Folgendes umfasst:
eine in den OSS-Sensor (40) integrierte Positionierungsführung (50);
wobei der OSS-Sensor (40) und die Positionierungsführung (50) mindestens teilweise in das Ernährungslumen eingeführt werden können oder eingeführt sind; und
wobei mindestens eines der Folgenden gilt:
die Ernährungssonde (30) umfasst zudem eine proximale OSS-Sensorverriegelung (60, 70), die das Einführen des OSS-Sensors (40) und der Positionierungsführung (50) in das Ernährungslumen fixiert; und
die Positionierungsführung (50) umfasst eine distale OSS-Sensorverriegelung (40), die das Einführen des OSS-Sensors (40) und der Positionierungsführung (50) in das Ernährungslumen fixiert.

3. Das OSS-Ernährungssondensystem gemäß Anspruch 1, wobei der OSS-Sensor (40) einen Lichtwellenleiter (41) umfasst; und wobei das System zudem einen Führungsdraht (51) umfasst, der den Lichtwellenleiter (41) einbettet, und der vollständig in das Ernährungslumen (32) der Ernährungssonde (30) eingeführt ist; wobei der Führungsdraht (51) einen Ballon (80) umfasst, der aufgeblasen werden kann oder ist, um das Einführen des Lichtwellenleiters (41) und des Führungsdrahts (51) in das Ernährungslumen (32) vorübergehend oder dauerhaft zu fixieren.

4. Das OSS-Ernährungssondensystem gemäß Anspruch 1, wobei die Ernährungssonde (30) zudem ein Navigationslumen umfasst; und
wobei der OSS-Sensor (40) im Navigationslumen positioniert werden kann oder ist.

5. Das OSS-Ernährungssondensystem gemäß Anspruch 1, das zudem Folgendes umfasst:
eine Schutzhülse, die in die Außenseite der Ernährungssonde (30) integriert ist,
wobei der OSS-Sensor (40) mindestens teilweise in die Schutzhülse eingeführt werden kann.

6. Das OSS-Ernährungssondensystem gemäß Anspruch 1, das zudem Folgendes umfasst:
eine Schutzhülse,
wobei die Ernährungssonde (30) und der OSS-Sensor (40) mindestens teilweise in die Schutzhülse eingeführt werden können.

7. Das OSS-Ernährungssondensystem gemäß Anspruch 1, das zudem Folgendes umfasst:
eine Navigationssteuerung, die als Reaktion auf das Generieren der Erfassungsdaten durch den OSS-Sensor (40) das Nachverfolgen der Form des mindestens einen Segments der OSS-Ernährungssonde (20) beim Positionieren im anatomischen Trakt steuert,
wobei das Nachverfolgen der Form der OSS-Ernährungssonde (20) beim Positionieren im anatomischen Trakt durch die Navigationssteuerung mindestens eines der beiden Folgenden umfasst:
die Navigationssteuerung, die die Anzeige der Form des mindestens einen Segments der OSS-Ernährungssonde (20) beim Positionieren im anatomischen Trakt steuert; und
die Navigationssteuerung, die das Ermitteln mindestens einer der OSS-Ernährungssonde (20) zugeordneten Metrik steuert, wenn diese im anatomischen Trakt positioniert ist.

8. Das OSS-Ernährungssondensystem gemäß Anspruch 7, wobei die Steuerung der Anzeige der Form des mindestens einen Segments der OSS-Ernährungssonde (20) beim Positionieren im anatomischen Trakt durch die Navigationssteuerung mindestens eines der Folgenden umfasst:
eine Anzeige der Form des mindestens einen Segments der OSS-Ernährungssonde (20) im Raum;
eine Anzeige der Form des mindestens einen Segments der OSS-Ernährungssonde (20) als Überlagerung auf einem Patientenbild, das mit der OSS-Ernährungssonde (20) erfasst wurde; und
eine Anzeige der Form des mindestens einen Segments der OSS-Ernährungssonde (20) als Überlagerung auf einem Patientenmodell, das mit der OSS-Ernährungssonde (20) erfasst wurde.

9. Das OSS-Ernährungssondensystem gemäß Anspruch 7, wobei das Steuern des Ermittelns der mindestens einen der im anatomischen Trakt positionierten OSS-Ernährungssonde (20) zugeordneten Metrik durch die Navigationssteuerung mindestens einen der folgenden Schritte umfasst:
Ermitteln der Einführlänge des mindestens einen Segments der der im anatomischen Trakt positionierten OSS-Ernährungssonde (20);
Vorhersagen der korrekten nasogastrischen Platzierung des mindestens einen Segments der im anatomischen Trakt positionierten Ernährungssonde (20); und
Messen physiologisch relevanter Parameter in Bezug auf den OSS-Sensor (40).

## Revendications

1. Système de sonde d'alimentation à détection de forme optique (ou « OSS »), comprenant :
une sonde d'alimentation (30) comprenant une lumière d'alimentation destinée à la canalisation d'un écoulement de fluide d'une extrémité proximale de la sonde d'alimentation (30) à une extrémité distale de la sonde d'alimentation (30) ;
un capteur OSS (40) destiné à la génération des données de détection informant d'une reconstruction de forme d'au moins un segment du capteur OSS (40) ;
dans lequel la sonde d'alimentation (30) et le capteur OSS (40) sont intégrés pour concevoir une sonde d'alimentation OSS (20) s'étendant entre l'extrémité proximale de la sonde d'alimentation (30) et une extrémité distale du capteur OSS (40) ; et
dans lequel les données de détection informent en outre sur une forme de l'au moins un segment de la sonde d'alimentation OSS (20) dérivée de l'intégration du capteur OSS (40) et de la sonde d'alimentation (30).

2. Système de sonde d'alimentation OSS selon la revendication 1, comprenant en outre :
un guide de positionnement (50) intégré au capteur OSS (40) ;
dans lequel le capteur OSS (40) et le guide de positionnement (50) sont au moins partiellement insérables ou partiellement insérés dans la lumière d'alimentation ; et dans lequel :
la sonde d'alimentation (30) comprend en outre un verrou proximal de capteur OSS (60, 70) utilisable pour fixer ou fixant l'insertion du capteur OSS (40) et du guide de positionnement (50) dans la lumière d'alimentation ; et/ou
le guide de positionnement (50) comprend un verrou distal de capteur OSS (40) utilisable pour fixer ou fixant l'insertion du capteur OSS (40) et du guide de positionnement (50) dans la lumière d'alimentation.

3. Système de sonde d'alimentation OSS selon la revendication 1, dans lequel le capteur OSS (40) comprend une fibre optique (41) ; le système comprenant en outre un fil de guidage (51) enrobant ladite fibre optique (41), et entièrement inséré dans la lumière d'alimentation (32) de la sonde d'alimentation (30), le fil de guidage (51) comprenant un ballonnet (80) gonflable ou gonflé pour fixer, de manière temporaire ou permanente, l'insertion de ladite fibre optique (41) et dudit fil de guidage (51) dans la lumière d'alimentation (32).

4. Système de sonde d'alimentation OSS selon la revendication 1,
dans lequel la sonde d'alimentation (30) comprend en outre une lumière de navigation ; et
dans lequel le capteur OSS (40) est peut être positionné ou est positionné dans la lumière de navigation.

5. Système de sonde d'alimentation OSS selon la revendication 1, comprenant en outre :
une gaine protectrice intégrée à un extérieur de la sonde d'alimentation (30),
dans lequel le capteur OSS (40) est au moins partiellement insérable dans la gaine protectrice.

6. Système de sonde d'alimentation OSS selon la revendication 1, comprenant en outre :
une gaine protectrice,
dans lequel la sonde d'alimentation (30) et le capteur OSS (40) sont au moins partiellement insérables dans la gaine protectrice.

7. Système de sonde d'alimentation OSS selon la revendication 1, comprenant en outre :
un dispositif de commande de navigation, sensible à une génération des données de détection par le capteur OSS (40), utilisable pour commander un suivi de la forme de l'au moins un segment de la sonde d'alimentation OSS (20), telle que positionnée dans un tractus anatomique,
dans lequel un suivi par le dispositif de commande de navigation de la forme de la sonde d'alimentation OSS (20) telle que positionnée dans le tractus anatomique, comprend :
le dispositif de commande de navigation utilisable pour commander un affichage de la forme d'au moins le segment de la sonde d'alimentation OSS (20), telle que positionnée dans le tractus anatomique ; et/ou le dispositif de commande de navigation utilisable pour commander une détermination d'au moins une métrique associée à la sonde d'alimentation OSS (20), telle que positionnée dans le tractus anatomique.

8. Système de sonde d'alimentation OSS selon la revendication 7, dans lequel la commande par le dispositif de commande de navigation de l'affichage de la forme d'au moins le segment de la sonde d'alimentation OSS (20), telle que positionnée dans le tractus anatomique, comprend :
un affichage de la forme d'au moins le segment de la sonde d'alimentation OSS (20) dans l'espace ; et/ou un affichage de la forme d'au moins le segment de la sonde d'alimentation OSS (20) en superposition sur une image de patient enregistrée avec la sonde d'alimentation OSS (20) ; et/ou
un affichage de la forme d'au moins le segment de la sonde d'alimentation OSS (20) en superposition sur un modèle de patient enregistré avec la sonde d'alimentation OSS (20).

9. Système de sonde d'alimentation OSS selon la revendication 7, dans lequel la commande par le dispositif de commande de navigation de la détermination de l'au moins une métrique associée à la sonde d'alimentation OSS (20), telle que positionnée dans le tractus anatomique, comprend :
une détermination d'une longueur d'insertion d'au moins le segment de la sonde d'alimentation OSS (20),
telle que positionnée à l'intérieur du tractus anatomique ; et/ou
une prédiction d'un placement correct d'au moins du segment de la sonde nasogastrique d'alimentation OSS (20), telle que positionnée dans le tractus anatomique ; et/ou
une mesure de paramètres physiologiquement pertinents associés au capteur OSS (40).
